# EUROPEAN PATENT SPECIFICATION

(11) **EP 3 871 629 B1**
(45) Date of publication and mention of the grant of the patent: **09.07.2025**
(21) Application number: 19876996.0
(22) Date of filing: 31.07.2019
(51) Int. Cl.: A61B 34/37, A61B 34/00, A61B 90/53, A61B 17/00

(54) **WEARABLE SURGICAL ROBOT ARM**
AM KÖRPER TRAGBARER CHIRURGISCHER ROBOTERARM
BRAS ROBOTIQUE CHIRURGICAL PORTABLE

(30) Priority: 26.10.2018 KR 20180129218
(43) Date of publication of application: 01.09.2021
(73) Proprietor: Yoon, Sam Youl, Seoul 05507 (KR)
(72) Inventor: YOON, So Jin, Suwon-si, Gyeonggi-do 16535 (KR)
(74) Representative: Canzler & Bergmeier Patentanwälte Partnerschaft mbB
(86) International application number: PCT/KR2019/009569
(87) International publication number: WO 2020/085624

(56) References cited:
- EP-A1- 3 278 748
- WO-A2-2011/002215
- CN-A- 108 113 757
- KR-A- 20110 003 229
- KR-A- 20120 068 097
- KR-A- 20120 068 097
- KR-A- 20150 115 764
- US-A1- 2005 215 983
- US-A1- 2009 005 638
- US-A1- 2009 005 638
- US-A1- 2015 164 601
- US-B2- 8 827 988

## Description

### 1. Field

The present disclosure relates to a wearable surgical robot arm, and more specifically, to a wearable surgical robot arm capable of proceeding a surgery through a surgical instrument that may be controlled by manipulation of an operator while the robot arm is fixed to an arm of the operator.

### 2. Background

Medically, a surgery refers to curing a disease by cutting, incising, or performing manipulation on skin, mucous membranes, and other tissues using a surgical instrument. Especially, an open surgery and laparoscopic surgery, in which the skin of a surgical site is cut open and then the internal organs therein are treated, formed, or removed, have problems of bleeding, adverse effects, patient's pain, scar, and precision of the surgery, and thus, in recent days, surgeries using robots are getting the spotlight as an alternative.

Conventional surgical robots, like the Da Vinci Surgical System developed by Intuitive Surgical, USA, consist of a master robot that generates and transmits a necessary signal by manipulation of a doctor and a slave robot that receives the signals from the master robot and directly applies a necessary manipulation for the surgery. Generally, the slave robot is installed in a surgery room while the master robot is installed in a manipulating room, that is separate from the surgery room, respectively, and the master robot and the slave robot are connected by wired or wireless methods to proceed remote operation. Examples of these systems are disclosed in US 2005/0215983 A1 and KR 2012 0068097 A.

However, conventional surgical robots have very large volumes and require considerable amount of space for installation, and the configuration of the device is complicated, and thus it takes a lot of time and cost in manufacturing, installing and learning the process. Especially, even in the case of a simple surgery, when using a conventional surgical robot, there is a problem that the robot surgery might become an inefficient method due to the time and cost being spent.

Further, when there is a need to use other surgical instruments during a procedure, a method is used where the position of the robot arm of the surgical robot currently being used is moved, and the surgical instrument is replaced by a surgical assistant and then moved back to the original position. In this case, however, the surgical assistant has to move the robot arm to a space in which the surgical instrument can be replaced, and thus it may cause unnecessary movement line of the robot arm. This is a problem in that unnecessary time is wasted considering that the nature of surgeries is to complete the procedure as quickly as possible. Further, there is a problem that additional workforce (1~2 surgical assistant nurses, 1 instrument delivery nurse) is necessary for a simple work of replacing the surgical instruments. The systems of the already mentioned US 2005/0215983 A1 and KR 2012 0068097 A allow automatic switching of the respective surgical instrument used by the robot arm. Furthermore, EP 3 278 748 A1, US 2009/0005638 A1 and US 8,827,988 B2 disclose hand operated surgical tools that allow switching of the respective surgical instrument without manual manipulation of the tool. The different instruments are stored in respective chambers within the tool.

Further, in a case where an unexpected emergency occurs to the patient during the surgery procedure using a robot, the doctor who was performing the surgery using the master robot in a remote location separated from the surgery room needs preparation time to directly participate in the surgery, thus the surgery time is inevitably delayed, which makes it impossible to immediately respond to the emergency situation. WO 2011/002215 A2 discloses a hand operated tool mounted on a robotic arm. US 2015/0164601 A1 discloses a laparoscopic tool configured to be fixed to an arm of a surgeon.

### SUMMARY

A purpose of the present disclosure is to resolve the problems of prior art, that is, to provide a wearable surgical robot arm that enables immediate response by the operator even when an unexpected emergency situation occurs, since a surgery is proceeded through a surgical instrument that is controlled by manipulation of the operator with the robot arm fixed to the arm of the operator.

Further, another purpose of the present disclosure is to provide a wearable surgical robot arm that may not only reduce the surgery time but also does not require additional workforce for replacing the surgical instrument, as the base mounted onto the operator's arm has a motion part onto which a plurality of surgical instruments are mounted, and replacement of the surgical instrument is made according to signals being input by the operator.

Further, another purpose of the present disclosure is to provide a wearable surgical robot arm that may prevent fatigue of the operator's arm from being increased by the self-weight of the robot arm, since the base mounted onto the operator's arm is supported against a structure by a multi-joint support arm.

The aforementioned purpose may be achieved according to the present disclosure by a wearable surgical robot arm comprising: a base configured to be fixed to an arm of an operator to move together with the arm of the operator; a motion part that has a plurality of accommodating grooves in which a surgical instrument is be mounted and that is moveably installed on the base such that one of the plurality of accommodating grooves is positioned in a first position; a shaft that is disposed such that the shaft moves forward or backward to selectively penetrate the accommodating groove positioned in the first position at a rear of the motion part; a first driver that moves the motion part such that one surgical instrument selected from the surgical instruments mounted in the plurality of accommodating grooves moves to the first position; a second driver that moves the shaft to move the surgical instrument positioned in the first position to a second position positioned in front of the first position; and a third driver that provides driving force to the surgical instrument that moved to the second position.

Here, it is desirable that the motion part is rotatably disposed on the base.

Further, it is desirable that the third driver is provided in the shaft.

Further, it is desirable that at a front end of the shaft, a driving wheel is provided for transmitting the driving force being provided from the third driver, and at a rear end of the surgical instrument, a following wheel is provided in a position correspond to the driving wheel to interlock with the driving wheel.

The base is provided with an inputter by which the operator may input a control signal.

The inputter includes a first inputter for inputting a control signal for controlling operations of the surgical instrument.

The inputter includes a second inputter for inputting a control signal for driving the first driver and the second driver.

The second inputter includes a voice inputter for recognizing voice of the operator, and a voice recognizer for generating a control signal for driving the first driver and the second driver according to the voice input through the voice inputter.

Further, it is desirable that the wearable surgical robot arm further includes a multi-joint support arm that supports the base in a moveable form.

According to the present disclosure, a wearable surgical robot arm is provided, that enables immediate response by the operator even when an unexpected emergency situation occurs, since a surgery is proceeded through a surgical instrument that is controlled by manipulation of the operator with the robot arm fixed to the arm of the operator.

Further, a wearable surgical robot arm is provided, that may not only reduce the surgery time but also does not require additional workforce for replacing the surgical instrument, as the base mounted onto the operator's arm has a motion part onto which a plurality of surgical instruments are mounted, and replacement of the surgical instrument is made according to signals being input by the operator.

Further, a wearable surgical robot arm is provided, that may prevent fatigue of the operator's arm being increased by the self-weight of the robot arm, since the base mounted onto the operator's arm is supported against a structure by a multi-joint support arm.

### BRIEF DESCRIPTION OF THE DRAWINGS

FIG. 1 is a schematic configuration view of a wearable surgical robot art of the present disclosure;
FIG. 2 is a perspective view of the wearable surgical robot arm of the present disclosure;
FIG. 3 is a function view illustrating operations of a motion part according to the wearable surgical robot arm of the present disclosure;
FIG. 4 is a function view illustrating operations of a shaft according to the wearable surgical robot arm of the present disclosure;
FIG. 5 is a perspective view illustrating a coupling site of the shaft and a surgical instrument according to the wearable surgical robot arm of the present disclosure; and
FIG. 6 is a view illustrating a modified example of the motion part according to the wearable surgical robot arm of the present disclosure.

### DETAILED DESCRIPTION

Prior to the detailed description, in numerous embodiments, it is to be noted that regarding components having the same configuration, explanation will be made with reference to a first embodiment as a representative using the same reference numerals, and in the rest of the embodiments, explanation will be made regarding configurations that are different from the first embodiment.

Hereinbelow, with reference to the drawings attached, detailed explanation will be made on a wearable surgical robot arm according to a first embodiment of the present disclosure.

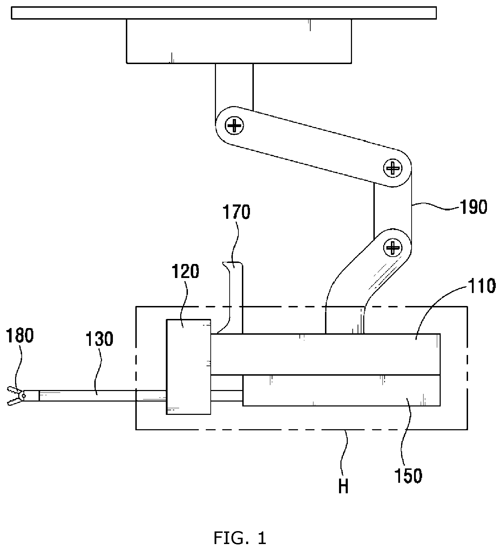

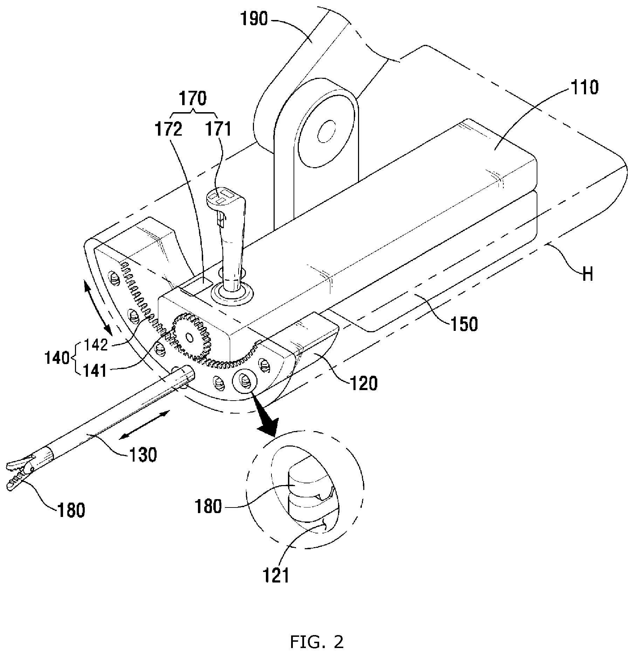

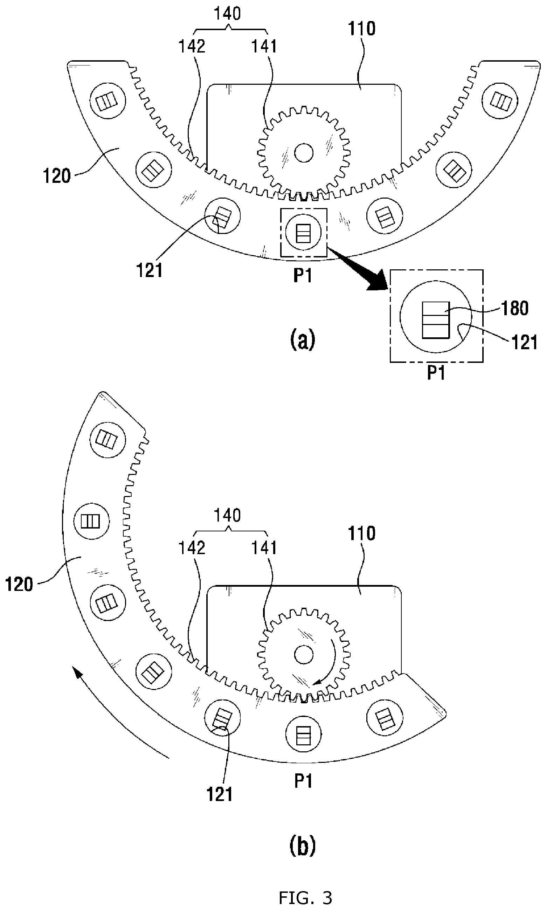

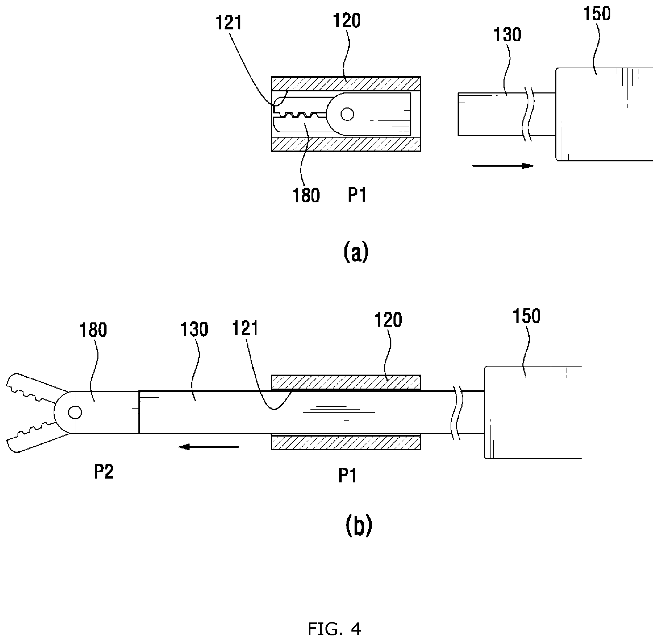

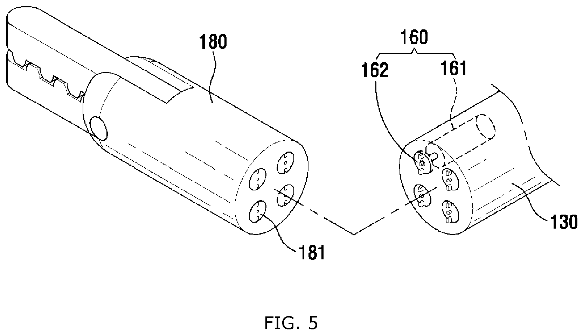

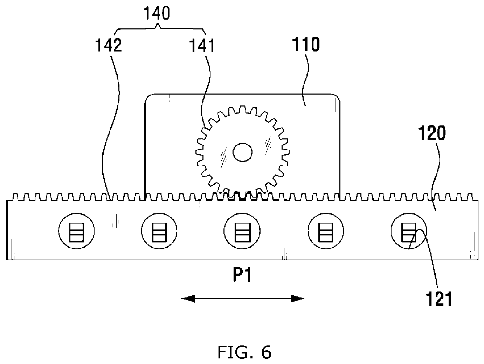

Of the attached drawings, FIG. 1 is a schematic configuration view of a wearable surgical robot art of the present disclosure, FIG. 2 is a perspective view of the wearable surgical robot arm of the present disclosure, FIG. 3 is a function view illustrating operations of a motion part according to the wearable surgical robot arm of the present disclosure, FIG. 4 is a function view illustrating operations of a shaft according to the wearable surgical robot arm of the present disclosure, FIG. 5 is a perspective view illustrating a coupling site of the shaft and a surgical instrument according to the wearable surgical robot arm of the present disclosure, and FIG. 6 is a view illustrating a modified example of the motion part according to the wearable surgical robot arm of the present disclosure.

A wearable surgical robot arm as that illustrated in the aforementioned drawings includes a base 110, a motion part 120, a shaft 130, a first driver 140, a second driver 150, a third driver 160, an inputter 170, a surgical instrument 180, and a multi-joint support arm 190.

Meanwhile, in order to prevent a complicated mechanical configuration from being exposed outside from a surgery room, it will be preferable to have a housing H for accommodating the base 110, the motion part 120, the first driver 140, and the second driver 150.

The base 110 is configured to be mounted onto an arm of an operator such as a surgeon, to move together with the operator's arm, and may have a band or a belt to be fixed to the arm, or it may be configured in a form to which the arm may be fitted.

The motion part 120 may be rotatably installed in the base 110 and may consist of arc-shaped cross sections. The motion part have a plurality of accommodating grooves 121 on the same radius about the center of rotation and a surgical instrument 180 may be mounted in the accommodating groove. The motion part may enable one of the plurality of accommodating grooves 121 to be positioned in a first position P1, according to the rotation position. Meanwhile, although not illustrated in the drawings, the motion part 120 may have an arc-shaped rail along the motion direction of the motion part 120, and the base 110 may have a block to be coupled to the rail so that rotation of the motion part 120 may be guided. Meanwhile, although in the present embodiment an example was explained that as the motion part 120 rotates, one of the plurality of accommodating grooves 121 moves to the first position P1, it will also be possible to configure such that as the motion part 120 moves left/right (or up/down), one of the plurality of accommodating grooves 121 moves to the first position P1 as illustrated in FIG. 6.

The shaft 130 is disposed on a same axis line as the accommodating groove 121 of the first position P1 at a rear of the motion part 120, and the shaft is supported against the base 110 so as to axially move back and forth. Meanwhile, although not illustrated in the drawings, at a front end of the shaft 130, a coupling instrument that may selectively fix or unfix a rear end of the surgical instrument 180 just as a gripper, may be provided.

The first driver 140 is for rotating the motion part 120 so that any one surgical instrument 180 selected from among the surgical instruments 180 mounted onto the plurality of accommodating grooves 121 can move to the first position P1, and the first driver may include a servo motor (not illustrated) provided in the base 110, a pinion 141 fixed to a driving axis of the servo motor, and an arc-shaped rake 142 that may be provided in the motion part 120 to interlock with the pinion 141. Meanwhile, in the present embodiment, explanation was made based on an example of rotating the motion part 120 using the pinion 141 and the rake 142, but there is no limitation thereto, and thus, various forms of driving means and power transmission mechanism that can control the rotation position of the moving part 120 may be applied.

The second driver 150 is for moving the shaft 130 forward and backward in an axial direction, and may be configured in the form of hydraulic or pneumatic cylinder, and the motion direction of the shaft 130 is set in a direction parallel with the rotation center axis of the motion part 120. In a case where the shaft 130 moves forward by driving of the second driver 150, the surgical instrument 180 disposed within the accommodating groove 121 of the first position P1 deviates from the accommodating groove 121, thus moving to a second position P2 located in front of the first position P1, and in a case where the shaft 130 moves backward by driving of the second driver 150, the surgical instrument 180 disposed in the second position P2 moves to the first position P1, and is inserted into the accommodating groove 121 of the motion part 120. Meanwhile, in the process of moving forward and backward of the shaft 130, it is desirable that the second driver 150 stops for a moment in the first position P1 and then move again for the sake of smooth coupling or uncoupling of the front end of the shaft 130 and the rear end of the surgical instrument 180.

The third driver 160 is for providing driving force to the surgical instrument 180 that is coupled to the front end of the shaft 130 and moved to the second position P2. The third driver 160 may include the servo motor 161, and a driving wheel 162 that is disposed at a front end of the shaft 130 and that rotates by the driving force being provided from the servo motor 161. The third driver 160 may be provided in plural number for various operations. Further, although not illustrated in the drawings, on a coupling surface of the shaft 130 and the surgical instrument 180, a terminal for mutual electrical connection for transmitting power source or signals may be further provided.

The inputter 170 is provided on the base 110 such that the operator can input controls signals for operation or replacement. The inputter 170 includes a first inputter 171 that may generate control signals for driving the third driver 160 and a second inputter 172 that may generate control signals for driving the first driver 140 and the second driver 150.

The first inputter 171 may include one or more of various input devices such as a joystick, glove, trigger-gun, and manual controller that may generate control signals for driving the third driver 160 according to the manipulation of the operator.

The second inputter 172 may include a voice inputter for recognizing the voice of the operator, and a voice recognizer that may generate control signals for driving the first driver 150 and the second driver 150 according to the voice input through the voice inputter. Meanwhile, although in the present embodiment, an example was explained where the first driver 140 and the second driver 150 are controlled by recognizing the voice of the operator, there is no limitation thereto, and thus it will also be possible to generate the control signals through a button that may be directly manipulated by the operator such as the first inputter or a sensor that may sense the operations of the operator.

The surgical instrument 180 may include a body part, an end effector that is provided at a front end of the body part, a following wheel 181 provided at a rear end of the body part, and a driving force transmission mechanism provided inside the body part to connect the following wheel 181 and the end effector, and the body part may have a joint. Such a surgical instrument 180 may be divided into forceps, electric cautery instruments, suture instruments, ultrasonic cutter instruments, washing and drainage instruments, etc., depending on the shape of the end effector, and may each be accommodated in the accommodating groove 121 of the motion part 120 by different type. The following wheel 181 is provided on the rear end of the surgical instrument 180 in a position corresponding to the driving wheel 162 of the third driver 160, and is configured to interlock with the driving wheel 162 to receive the driving force being provided from the third driver 160.

The multi-arm support arm 190 is connected to the base 110 at one end, and the other end is connected to the structure such as a wall, ceiling, and bottom, to support the base, and may include a gravity compensation mechanism for compensating the gravity by self-weight of the robot arm.

Hereinbelow, operations of the first embodiment of the wearable surgical robot arm will be explained.

The base 110 may be fixed to the arm of the operator and move together with the arm. The base 110 is supported against the structure such as the ceiling, wall and bottom inside the surgery room by the multi-joint support arm 190. Therefore, in the surgery room, with the operator wearing the surgical robot arm, the operator may manipulate the first inputter 171 provided in the base 110 to control the surgical instrument 180 mounted onto the robot arm, thereby proceeding with the surgery, and input a voice to the second inputter 172 to easily replace the surgical instrument 180 mounted onto the robot arm. Especially, since the operator may take off the robot arm and treat the surgical site directly with hands, when a dangerous situation occurs, immediate response is also possible.

The motion part 120 is rotatably installed in the base 110 so that it may be rotated by the first driver 140, and in the plurality of accommodating grooves 121 formed in the motion part 120, different types of surgical instruments 180 are inserted, respectively. When the operator makes a verbal instruction using voice to use a surgical instrument 180 that the operator wants to use during the surgery, the second driver 150 is driven backward to move the shaft 130 backward, thereby positioning the surgical instrument 180 of the second position P2 that is in use to the accommodating groove 121 of the first position P1, and the first driver 140 is driven to move the accommodating groove 121 to which the surgical instrument 180 is inserted corresponding to the corresponding voice instruction made, to the first position P1, and then the second driver 150 is driven forward to drive the shaft 130 forward, thereby moving the surgical instrument 180 disposed in the first position P1 to the second position P2.

As aforementioned, according to the present embodiment, replacement of the surgical instrument 180 may be made with only the voice of the operator, and thus it is not only possible to reduce the surgery time, but also additional workforce for replacing the surgical instrument 180 is not required. Further, since the surgeon may pre-configure a combination of the instruments necessary for the scheduled surgery, and dispose the accommodating groove 121 of the motion part 120 according to the order of use, the time spent on replacing the surgical instrument 180 may be minimized.

As aforementioned, in the surgical instrument 180 disposed in the second position P2, operation of the end effector is controlled as the operator manipulates the first inputter 171, and thus controlling the driving of the third driver 160. Therefore, a surgery may be proceeded using the end effector of the surgical instrument 180.

Especially, in a case of providing the first inputter 171 in a similar form as a manipulating part of a laparoscopic surgical instrument, a surgery may be proceeded in a form that is similar to a laparoscopic surgery, with the robot arm of the present embodiment mounted onto the arm of the operator, and thus, an operator experienced in laparoscopic surgery may use the robot arm of the present embodiment intuitively.

The scope of the present invention is not limited to the above-described embodiments, but may be implemented in various forms within the scope of the attached claims.

## Claims

1. A wearable surgical robot arm comprising:
a base (110) configured to be fixed to an arm of an operator to move together with the arm of the operator;
a motion part (120) that has a plurality of accommodating grooves in (121) which a surgical instrument is be mounted and that is moveably installed on the base such that one of the plurality of accommodating grooves is positioned in a first position;
a shaft (130) that is disposed such that the shaft moves forward or backward to selectively penetrate the accommodating groove positioned in the first position at a rear of the motion part;
a first driver (140) that moves the motion part such that one surgical instrument selected from the surgical instruments mounted in the plurality of accommodating grooves moves to the first position;
a second driver (150) that moves the shaft to move the surgical instrument positioned in the first position to a second position positioned in front of the first position; and
a third driver (160) that provides driving force to the surgical instrument that moved to the second position,
wherein the base (110) is provided with an inputter (170) by which the operator inputs a control signal, and the inputter (170) includes a first inputter (171) for inputting a control signal for driving the third driver to control operations of the surgical instrument and a second inputter (172) for inputting a control signal for driving the first driver and the second driver to replace the surgical instrument, and
wherein the second inputter (172) includes a voice inputter for recognizing voice of the operator and a voice recognizer for generating a control signal for driving the first driver and the second driver according to the voice input through the voice inputter such that a replacement of the surgical instrument is made by only the voice of the operator.

2. The wearable surgical robot arm according to claim 1,
wherein the motion part (120) is rotatably disposed on the base (110).

3. The wearable surgical robot arm according to claim 1,
wherein the third driver (160) is provided in the shaft (130).

4. The wearable surgical robot arm according to claim 1,
wherein at a front end of the shaft (130), a driving wheel (162) is provided for transmitting the driving force being provided from the third driver, and at a rear end of the surgical instrument (180), a following wheel (181) is provided in a position correspond to the driving wheel to interlock with the driving wheel (162).

5. The wearable surgical robot arm according to claim 1,
further including a multi-joint support (190) arm that supports the base (110) in a moveable form.

## Patentansprüche

1. Tragbarer chirurgischer Roboterarm, umfassend:
eine Basis (110), die konfiguriert ist, um an einem Arm eines Bedieners befestigt zu werden, um sich zusammen mit dem Arm des Bedieners zu bewegen;
ein Bewegungsbauteil (120), das eine Vielzahl von Aufnahmenuten (121) aufweist, in denen ein chirurgisches Instrument montiert werden soll, und das beweglich auf der Basis installiert ist, so dass eine der Vielzahl von Aufnahmenuten in einer ersten Position positioniert ist;
einen Schaft (130), der so angeordnet ist, dass sich der Schaft vorwärts oder rückwärts bewegt, um selektiv in die Aufnahmenut einzudringen, die in der ersten Position an einer Rückseite des Bewegungsbauteils positioniert ist;
einen ersten Antrieb (140), der das Bewegungsbauteil so bewegt, dass sich ein chirurgisches Instrument, das aus den chirurgischen Instrumenten ausgewählt ist, die in der Vielzahl von Aufnahmenuten montiert sind, in die erste Position bewegt;
einen zweiten Antrieb (150), der den Schaft bewegt, um das chirurgische Instrument, das in der ersten Position positioniert ist, in eine zweite Position zu bewegen, die vor der ersten Position positioniert ist; und
einen dritten Antrieb (160), der eine Antriebskraft für das chirurgische Instrument bereitstellt, das sich in die zweite Position bewegt hat,
wobei die Basis (110) mit einem Eingabemittel (170) versehen ist, durch das der Bediener ein Steuersignal eingibt, und das Eingabemittel (170) ein erstes Eingabemittel (171) zum Eingeben eines Steuersignals zum Antreiben des dritten Antriebs zum Steuern von Operationen des chirurgischen Instruments und ein zweites Eingabemittel (172) zum Eingeben eines Steuersignals zum Antreiben des ersten Antriebs und des zweiten Antriebs zum Ersetzen des chirurgischen Instruments umfasst, und
wobei das zweite Eingabemittel (172) ein Spracheingabemittel zum Erkennen der Stimme des Bedieners und einen Spracherkenner zum Erzeugen eines Steuersignals zum Antreiben des ersten Antriebs und des zweiten Antriebs gemäß der Spracheingabe durch das Spracheingabemittel umfasst, so dass ein Ersetzen des chirurgischen Instruments nur durch die Stimme des Bedieners erfolgt.

2. Tragbarer chirurgischer Roboterarm nach Anspruch 1,
wobei das Bewegungsbauteil (120) drehbar auf der Basis (110) angeordnet ist.

3. Tragbarer chirurgischer Roboterarm nach Anspruch 1,
wobei der dritte Antrieb (160) in dem Schaft (130) vorgesehen ist.

4. Tragbarer chirurgischer Roboterarm nach Anspruch 1,
wobei an einem vorderen Ende des Schafts (130) ein Antriebsrad (162) zum Übertragen der Antriebskraft vorgesehen ist, die von dem dritten Antrieb bereitgestellt wird, und an einem hinteren Ende des chirurgischen Instruments (180) ein Folgerad (181) in einer Position vorgesehen ist, die dem Antriebsrad entspricht, um mit dem Antriebsrad (162) ineinanderzugreifen.

5. Tragbarer chirurgischer Roboterarm nach Anspruch 1,
ferner umfassend einen Mehrgelenkstützarm (190), der die Basis (110) in einer beweglichen Form stützt.

## Revendications

1. Bras de robot chirurgical portable comprenant :
une base (110) configurée pour être fixée à un bras d'un utilisateur pour se déplacer conjointement avec le bras de l'utilisateur ;
une partie de mouvement (120) qui a une pluralité de rainures de réception (121) dans lesquelles un instrument chirurgical doit être monté et qui est installée de manière mobile sur la base de sorte que l'une de la pluralité de rainures de réception est positionnée dans une première position ;
une tige (130) qui est disposée de sorte que la tige se déplace vers l'avant ou vers l'arrière pour pénétrer sélectivement dans la rainure de réception positionnée dans la première position à l'arrière de la partie de mouvement ;
un premier entraînement (140) qui déplace la partie de mouvement de sorte qu'un instrument chirurgical sélectionné parmi les instruments chirurgicaux montés dans la pluralité de rainures de réception se déplace vers la première position ;
un deuxième entraînement (150) qui déplace la tige pour déplacer l'instrument chirurgical positionné dans la première position vers une deuxième position positionnée devant la première position ; et
un troisième entraînement (160) qui fournit une force d'entraînement à l'instrument chirurgical qui s'est déplacé vers la deuxième position,
dans lequel la base (110) est pourvue d'un moyen d'entrée (170) par lequel l'utilisateur entre un signal de contrôle, et le moyen d'entrée (170) comprend un premier moyen d'entrée (171) pour entrer un signal de contrôle pour entraîner le troisième entraînement pour contrôler les opérations de l'instrument chirurgical et un deuxième moyen d'entrée (172) pour entrer un signal de contrôle pour entraîner le premier entraînement et le deuxième entraînement pour remplacer l'instrument chirurgical, et
dans lequel le deuxième moyen d'entrée (172) comprend un moyen d'entrée vocal pour reconnaître la voix de l'utilisateur et un dispositif de reconnaissance vocal pour générer un signal de contrôle pour entraîner le premier entraînement et le deuxième entraînement selon l'entrée vocale par l'intermédiaire du moyen d'entrée vocal de sorte qu'un remplacement de l'instrument chirurgical est effectué uniquement par la voix de l'utilisateur.

2. Bras de robot chirurgical portable selon la revendication 1,
dans lequel la partie de mouvement (120) est disposée de manière rotative sur la base (110).

3. Bras de robot chirurgical portable selon la revendication 1,
dans lequel le troisième entraînement (160) est prévu dans la tige (130).

4. Bras de robot chirurgical portable selon la revendication 1,
dans lequel à une extrémité frontale de la tige (130), une roue d'entraînement (162) est prévue pour transmettre la force d'entraînement fournie par le troisième entraînement, et à une extrémité arrière de l'instrument chirurgical (180), une roue suiveuse (181) est prévue dans une position correspondant à la roue d'entraînement pour s'interverrouiller avec la roue d'entraînement (162).

5. Bras de robot chirurgical portable selon la revendication 1,
comprenant en outre un bras de support à articulations multiples (190) qui supporte la base (110) sous une forme mobile.
